# EUROPÄISCHE PATENTANMELDUNG

(11) **EP 2 857 838 A2**
(43) Veröffentlichungstag der Anmeldung: **08.04.2015**
(21) Anmeldenummer: 14187534.4
(22) Anmeldetag: 02.10.2014
(51) Int. Cl.: G01N 33/543

(54) **Verfahren zur immunomagnetischen Abtrennung von Analyten aus einer komplexen Probenmatrix**

(30) Priorität: 02.10.2013 EP 13187037
(71) Anmelder: FZMB GmbH Forschungszentrum für Medizintechnik und Biotechnologie, 99947 Bad Langensalza (DE)
(72) Erfinder: Miethe, Peter, 99947 Bad Langensalza (DE); Platz, Sabine, 99947 Bad Langensalza (DE)
(74) Vertreter: Von Kreisler Selting Werner - Partnerschaft von Patentanwälten und Rechtsanwälten mbB

(57) **Zusammenfassung**

Verfahren zur Abtrennung eines Analyten aus einer komplexen Probe mittels magnetischer Partikel, welche eine ferromagnetische Legierung aus Mangan/ Zink Ferrit enthalten oder daraus bestehen mit den folgenden Schritten:
a) Bereitstellung und Aufnahme der Probe in einer Flüssigkeit,
b) Bereitstellung von ferromagnetischen Partikeln, welche eine ferromagnetische Legierung aus Mangan/ Zink Ferrit enthalten oder daraus bestehen, mit einer mittleren Größe von 0,3-50 µm als magnetischer Partikel, an deren Oberfläche ein den Analyt bindender immobilisierter Ligand angeordnet ist,
c) in Kontakt bringen der Probe mit den ferromagnetischen Partikeln,
d) Einwirken lassen der in der Probe befindlichen ferromagnetischen Partikeln zum Binden des Analyten,
e) Anlegen eines Magnetfeldes,
f) Abtrennung der in dem Magnetfeld festgehaltenen ferromagnetischen Partikeln.

SEQUENCE LISTING

<110>  fzmb GmbH Forschungszentrum für Medizintechnik und
       Biotechnologie

<120>  Verfahren zur Abtrennung von Analyten aus einer komplexen
       Probenmatrix

<130>  141859EP

<150>  13187037.0
<151>  2013-10-02

<160>  4

<170>  PatentIn version 3.3

<210>  1
<211>  21
<212>  DNA
<213>  artificial

<220>
<223>  Primer

<400>  1
acaaaacata tgctggacca a                                                21


<210>  2
<211>  26
<212>  DNA
<213>  artificial

<220>
<223>  Primer

<400>  2
cttaagtgta atgagatcca tcaaat                                           26


<210>  3
<211>  20
<212>  DNA
<213>  artificial

<220>
<223>  Primer

<400>  3
gaacgtgttt ccgtgcgtaa                                                  20


<210>  4
<211>  20
<212>  DNA
<213>  artificial

<220>
<223>  Primer

<400>  4
tcaaattagc ggaggcttcc                                                  20

## Beschreibung

Gegenstand der vorliegenden Erfindung ist ein Verfahren zur Abtrennung von Analyten aus einer Probe und eine Vorrichtung zur Durchführung des Verfahrens.

Bei einer Vielzahl von analytischen Fragestellungen liegt der Analyt in einer komplexen Probenmatrix vor und muss in einem Probenextraktionsschritt, der dem eigentlichen Analyseverfahren vorgeschaltet ist, abgetrennt werden. Typische Fälle sind die Extraktion von Mikroorganismen, Viren, Toxinen aus klinischen-, Umwelt-, und Lebensmittelproben.

Dazu werden Flüssigextraktionen, Festphasenextraktionen auf Säulen und Partikeldispersionen eingesetzt. Eine besonders vorteilhafte Form der Partikelextraktion ist die Magnetpartikelextraktion.

Zur Magnetseparation werden Magnetpartikel mit verschiedenen Oberflächenmodifikationen, insbesondere mit ionischen, hydrophoben oder bioaffinen Liganden (Lektine, Antikörper, Rezeptoren, Enzymsubstrate) eingesetzt. Dabei weisen die bioaffinen Liganden, die in Form der immunomagnetische Separation (IMS) auf Basis von Antikörpern das größte anwendungstechnische Potential besitzt, die größte Selektivität auf. Im Markt sind im Wesentlichen paramagnetische Partikel im Größenbereich von 1-4 µm verfügbar, bei denen paramagnetische Nanopartikel kleiner 30 nm in der Polymermatrix (Latex, Silikate) dispergiert sind. Die Extraktion aus komplexen Medien wie Lebensmitteln mit diesen Partikeln ist nachteilig wegen einer schlechten Abtrennbarkeit und komplexen Handhabung dieser Partikeln. Darüber hinaus ist auch der recht hohe Preis dieser Partikel dafür verantwortlich, dass Trennverfahren nicht ökonomisch sind. Daher beschränkt sich der praktische Einsatz nur auf den Forschungsbereich und kleine Probenvolumina.

### Stand der Technik

Die immunomagnetische Separation (IMS) wurde bereits Anfang der 1980er Jahre von Mattingly für die Isolation von *Salmonella* aus Lebensmitteln und Fäzes eingesetzt¹. Seitdem sind eine Reihe von Anwendungen entwickelt worden, zu denen die Konzentrierung von Zellen des Immunsystems, von Bakterien und Viren sowie die Isolation von Proteinen und DNA gehören^{2,3,4,5,6}. Die Magnetseparation hat sich insbesondere im Laboreinsatz als ein geeignetes Hilfsmittel zur Extraktion von unterschiedlichsten Zellen aus kleinen Volumina etabliert⁷. Derzeit gibt es nur vereinzelte Extraktionsansätze, die eine Aufkonzentrierung von Zellen aus großen Volumina vorsieht. Entsprechend ist auch die kommerziell erhältliche Extraktionstechnik fast ausschließlich auf wässrige und niedrig viskose Ausgangsmischungen von wenigen Millilitern ausgelegt⁸.

Die Anwendungsschritte einer Analyse, in der Magnetpartikel zum Einsatz kommen, sind einfach durchzuführen. Es erfolgt eine Inkubation der Magnetpartikel in dem Gemisch, aus dem extrahiert werden soll. Die Magnetpartikel binden an das Target (z.B. Bakterien, Viren, Toxine, Nukleinsäure). Bei dieser Affinitätsbindung, die bei Verwendung von Antikörpern auch als Immonucapture *bezeichnet wird,* wird das Zielmolekül oder Partikel über die spezielle Beschichtung der Magnetpartikel spezifisch gebunden. Mittels eines Magneten werden die Partikel dann an der Wand des Reaktionsgefäßes fixiert und der Überstand verworfen. Es kann eine Waschung der gebundenen Partikel erfolgen, um z.B. Inhibitoren einer nachfolgenden Nachweisreaktion zu entfernen. Anschließend erfolgt die Resuspendierung in einem neuen Puffer, die gebundenen Partikel können anschließend z.B. für eine PCR direkt lysiert werden, um die DNA freizusetzten. Für kleine Flüssigkeitsvolumina bis zu 5 ml sind Verfahren etabliert, die mit guten Ergebnissen im Labor eingesetzt werden^{9,10,11}.

Analog kann auch mit anderen Analyten wie beispielsweise Toxinen verfahren werden. Der Nachweis kann dabei entweder direkt am Partikel zur Zugabe eines Konjugates, das zur Bildung eines gut detektierbaren Sandwichkomplexes führt oder nach Dissoziation vom Magnetpartikel in einem instrumentellen analytischen Verfahren, wie der Massenspektroskopie erfolgen. Verschiedene Autoren haben auch die Anwendbarkeit der magnetischen Extraktion in Verbindung mit unterschiedlichen Detektionssystemen in Form von Bioassays beschrieben. Einen Überblick über die Nutzung von PCR-, Elektroden- und ELISA-basierter Detektion mit Magnetic Beads geben HAUKANES UND KVAM (1993)¹².

Im Allgemeinen werden paramagnetische Partikel verwendet.

Eine Übersicht über die wichtigsten Anbieter kommerzieller paramagnetischer Partikel ist im Folgenden wiedergegeben:
http://www.turbobeads.com: TurboBeads sind metallische Nanopartikel, welche mit einer extreme dünnen Graphitschicht (∼ 2 nm) beschichtet sind. Diese wird chemisch modifiziert und dadurch funktionalisiert. Die Größe dieser Partikel beträgt 30 nm. [TurboBeads Llc., Magdalenenstrasse 9, CH-8050 Zürich] http://www.chemicell.com: SiMAG Partikel sind magnetische Silica Beads mit superparamagnetischen Eigenschaften. Die Partikel haben entweder eine hochporöse oder eine nicht-poröse Oberfläche und sind zwischen 0,5 - 1 µm im Durchmesser. Es werden verschiedene Oberflächen-Modifikationen angeboten. [chemicell GmbH, Eresburgstrasse 22-23, 12103 Berlin]
http://www.microparticles.de: Es werden magnetische Partikel im Durchmesserbereich von 300 nm-50 µm angeboten. Die Matrix der Partikel besteht entweder aus Polystyrol oder Silica mit homogen inkorporiertem nanoskaligem Eisenoxid. Neben unmodifizierten magnetischen Partikeln (PS-MAG und SiO₂-MAG) und magnetischen Partikeln mit COOH-Modifizierung (PS-MAG-COOH und SiO₂-MAG-COOH werden auch magnetische Partikel mit Streptavidin-Funktionalisierung an (PS-MAG-SA und SiO₂-MAG-SA) angeboten. [microParticles GmbH, Volmerstr. 9A, H3.5.1, D-12489 Berlin]
http://www.chemagen.com: Diese superparamagnetischen Partikel bestehen aus einer Polyvinylalkohol-Matrix, welche nanopartikulären Magnetit enthält. Die Oberfläche wurde carboxyliert um mit den bekannten Methoden verschiedene Liganden zu koppeln. Es werden Partikel im Größenbereich von 0,5 - 3 µm angeboten. [PerkinElmer chemagen Technologie GmbH, Arnold-Sommerfeld-Ring 2, 52499 Baesweiler].
http://www.carlroth.de: Roti®-MagBeads besteht aus gleichmäßigen, superparamagnetischen Silicabeads. Die Größe beträgt durchschnittlich 1 µm. Der magnetische Anteil aus Eisen(II/III)oxid ist in Form kleiner Partikeln im Nanometermaßstab in Siliziumoxid eingebettet. Die Oberfläche besteht aus Siliziumoxid mit Oberflächenmodifikatonen wie Streptavidin, COOH, Protein A oder Protein G. [Carl Roth GmbH + Co. KG, Schoemperlenstr. 3-5, 76185 Karlsruhe].
http://de-de.invitrogen.com/site/d/de/home/brands/Product-Brand/Dynal/Dynabeads-Types-and-Uses.html: Dynabeads werden in verschiedenen Größen und mit verschiedenen Oberflächenmodifikationen angeboten. Alle angebotenen Partikel sind superparamagnetische Partikel. Typische Dynabeads mit Streptavidin-Beschichtung sind 1 µm im Durchmesser. [Life Technologies GmbH, Frankfurter Straße 129B, 64293 Darmstadt]

Die paramagnetische Separation zur Abtrennung von den Mikroorganismen wird außerdem in den folgenden Publikationen näher erläutert:
Olsvik O, Popovic T, Skjerve E, Cudjoe KS, Hornes E, Ugelstad J, Uhlen M (1994): Magnetic separation techniques in diagnostic microbiology. Clin Microbiol Rev. 7(1): 43-54
Mullane NR, Murray J, Drudy D, Prentice N, Whyte P, Wall PG, Parton A, Fanning S. (2006): Detection of Enterobacter sakazakii in dried infant milk formula by cationic-magnetic-bead capture. Appl. Environ Microbiol. 72(9): 6325-30.
Weitere Informationen zum Stand der Technik ergeben sich aus den folgenden Druckschriften.
Birkmeyer et al.(Clin. Chem. 33/9, 1543-1547, (1987)) offenbart die Verwendung von Chromdioxid Partikeln als Trägermaterial in immunologischen Tests.
US 2,885,366 A offenbart Partikel aus einem festen Kern und einer Umhüllung aus amorphen Siliziumdixid.
Widjojoatmodjo et al. (Eur. J. Clin. Microbiol. Infect. Dis., November 1991, p. 935-938) offenbart eine Methode zur Detektion von Salmonella mittels Magnetic Immuno PCR Assay. Dazu werden Partikel aus Chromdioxid mit monoclonalen Antikörpern beschichtet und zur schnellen Detektion von Salmonellen verwendet.
Widjojoatmodjo et al. (Journal of Clinical Microbiology, Dec. 1992, p. 3195-3199) offenbart ein auf MIPA basierendes Verfahren zur Detektion von Salmonellen in Stuhlproben.
Fluit et al. (Applied and Enviromental Microbiology, May 1993, p. 1289-1293) offenbart eine Methode zur Detektion von Listeria monocytogenes in Käse. Die verwendeten Partikel sind aus Chromdioxid und mit Ziegen Antikörpern beschichtet.
Fluit et al. (Applied and Enviromental Microbiology, May 1993, p. 1342-1346) offenbart eine Methode zur Detektion von Salmonellen in Hühnerfleisch. Die verwendeten Partikel sind aus Chromdioxid und mit Ziegen Antikörpern beschichtet.
"Ferromagnetic Microparticles and Nanoparticles-Spherotech" in (http://www.spherotech.com/fer par.htm) vom 12.03.2013 offenbart Größen-und Konzentrationsangaben zu ferromagnetischen Partikeln aus Chromdioxid.
Von Schönfeld et al. (Biology of Reproduction 61, 582-589 (1999)) offenbart eine Methode zur Isolierung größerer Mengen Spermatogonia.
Duodu et al. (Food Anal. Methods (2009) 2:23-29) offenbart eine Methode zur Immunomagnetischen Separation in der Probenvorbereitung in Verbindung mit echtzeit PCR zur Detektion und mengenmäßigen Bestimmung von Listeria monocytogenes in Räucherlachs.

Generell gilt, dass die beschriebenen paramagnetischen Partikeln durch ein magnetisches Feld angezogen werden, aber keine permanente Magnetisierung zeigen, sodass sie nach Abschalten des Feldes durch einfaches Schütteln wieder homogen in Lösung überführt werden können¹³.

Aus komplexen Proben, wie Lebensmitteln, Umweltproben, Gewebeproben, Vollblutproben, lassen sich die paramagnetischen Partikel aufgrund unzureichender Magnetkräfte jedoch oft nur ungenügend abtrennen

Versuche mittels paramagnetischen Partikeln eine immunomagnetische Separation aus Lebensmittelproben wie Rohmilch und Hackfleisch-Peptonwasser-Gemisch aus Volumina von 50 ml bis 250 ml durchzuführen haben gezeigt, dass paramagnetische Partikel sich nicht oder nur ungenügend für eine Extraktion aus einer viskösen und fetthaltigen Matrix eignen. Paramagnetische Partikel werden von Fetttropfen (z.B. bei Rohmilch der Sahnefraktion oder den Fettbestandteilen des Hackfleisches) derart umschlossen, das eine Rückgewinnung der Partikel nicht oder nur sehr ungenügend möglich ist.

Ein der Erfindung zu Grunde liegendes Problem liegt mithin darin, die genannten Nachteile des Standes der Technik zu überwinden und ein Verfahren anzugeben, das eine einfache Trennung von Analyten aus komplexen Proben ermöglicht.

Dieses Problem wird erfindungsgemäß gelöst durch ein Verfahren mit den Merkmalen des Patentanspruchs 1.

Das erfindungsgemäße Verfahren zur Abtrennung eines Analyten aus einer komplexen Probe mittels magnetischer Partikel, welche eine ferromagnetische Legierung aus Mangan/ Zink Ferrit enthalten oder daraus bestehen, weist die folgenden Schritte auf:
a) Bereitstellung und Aufnahme der Probe in einer Flüssigkeit,
b) Bereitstellung von ferromagnetischen Partikeln welche eine ferromagnetische Legierung aus Mangan Mangan/ Zink Ferrit enthalten oder daraus bestehen mit einer mittleren Größe von 0,3-50 µm als magnetischer Partikel, an deren Oberfläche ein den Analyt bindender immobilisierter Ligand angeordnet ist,
c) in Kontakt bringen der Probe mit den ferromagnetischen Partikeln,
d) Einwirkenlassen der in der Probe befindlichen ferromagnetischen Partikeln zum Binden des Analyten,
e) Anlegen eines Magnetfeldes,
f) Abtrennung der in dem Magnetfeld festgehaltenen ferromagnetischen Partikeln.

Das im erfindungsgemäßen Verfahren verwendbare ferromagnetische Partikel ist insbesondere monolithisch, d.h. es besteht aus einem einheitlichen magnetischen Material und ist nicht ein paramagnetisches Partikel, das in einer anderen Festphase dispergiert ist.

Gemäß des erfindungsgemäßen Verfahrens besteht oder umfasst das Partikel aus einer ferromagnetischen Legierung insbesondere Mangan/Zink Ferrit. Überraschenderweise zeigen Partikel aus Mangan/Zink Ferrit im erfindungsgemäßen Verfahren verbesserte magnetische Eigenschaften, d.h. auch in einem matrixreichen Medium, z.B. Fett, Polysaccharide oder Eiweiße, wird die magnetische Wirkung der Partikel nicht wesentlich eingeschränkt. Vergleichbare Partikel, z.B. aus ferromagnetischen Chromdioxid, werden beispielsweise durch fettreiche Medien stärker abgeschirmmt und in ihrer auf Magnetismus beruhenden Trennwirkung eingeschränkt.

Der im erfindungsgemäßen Verfahren einsetzbare ferromagnetische Partikel kann eine Größe d₅₀ von 0,3 bis 30 µm, vorzugsweise 6-8 µm aufweisen.

In einer weiteren Ausführungsform des erfindungsgemäßen Verfahrens kann der den Analyten bindende immobilisierte Ligand beim in Kontakt bringen mit dem Analyten mit diesem eine biospezifische Wechselwirkung ausbilden. Der Begriff "biospezifische Wechselwirkung" ist für den Fachmann klar. Er versteht darunter unter anderem eine Wechselwirkung, insbesondere hinreichend feste Bindung, wie sie beim in Kontakt bringen von Rezeptoren und ihren Liganden stattfindet. Dazu gehören beispielsweise Affinitätswechselwirkungen wie Rezeptor/Ligand Wechselwirkungen, wie zum Beispiel Wechselwirkungen zwischen Epitop und Idiotyp (Antikörper/Antigen), Lektin/Glycoprotein, Protein A/IgG, Biotin/Streptavidin, aber auch Enzym/Substrat Wechselwirkungen. Typischerweise kann im erfindungsgemäßen Verfahren der den Analyten bindende immobilisierte biospezifische Ligand ein Oligo- oder Polypeptid, insbesondere ein Antikörper, ein Antikörperfragment, z. B. scFab, eine Bindungsdomäne eines Enzyms, eine Nukleinsäurebindungsprotein, oder ein Lektin sein. Insbesondere kann der den Analyt bindende immobilisierte biospezifische Ligand ein Oligo- oder Polysaccharid, ein Oligonukleotid, insbesondere eine DNA Sonde sein. Es ist auch möglich eine ionische Gruppe, insbesondere eine Carboxy-, Sulfon-, Sulfat- Aminogruppe zur Bindung eines Analyten einzusetzen, wobei sich beim in Kontakt bringen eine ionische Wechselwirkung mit diesem ausbildet.

Im erfindungsgemäßen Verfahren kann der Ligand insbesondere über eine Zwischenschicht bestehend aus einem quervernetzen Protein, einem polykondensierten Siloxan oder einem Polysacharid, insbesondere einem Aminozellulosecarbamat an den ferromagnetischen Partikel gebunden sein. Besonders vorteilhaft ist die Beschichtung mit Aminozellulosecarbamat entsprechend DE102013005184 mit dem Titel "Verfahren zur Funktionalisierung einer Oberfläche" und die Kopplung der Liganden über einen Dicarbonsäurelinker.

In einer bevorzugten Ausführungsform des erfindungsgemäßen Verfahrens schließt sich an den Schritt f) ein weiterer Schritt an, bei dem der Analyt oder Teile des Analyten vom Partikel in eine abgetrennte Fraktion dissoziiert werden, und für den Nachweis die abgetrennte Fraktion eingesetzt wird.

Gemäß einer weiteren Ausführungsform des erfindungsgemäßen Verfahrens erfolgt der Nachweis des Analyten am ferromagnetischen Partikel durch Zugabe eines detektierbaren Konjugates, das mit dem Analyten eine spezifische Bindung eingeht.

Der Analyt im erfindungsgemäßen Verfahren kann ein Mikroorganismus, Teil eines Mikroorganismus, ein Virus oder ein Toxin aus einer klinischen Probe, einer Umwelt-, Lebensmittelprobe oder einer Probe aus einem pharmazeutischen Rohstoff oder Wirkstoffes sein.

In noch einer weiteren Ausführungsform des erfindungsgemäßen Verfahrens kann der Analyt eine in der Probe frei vorhandene RNA oder DNA sein.

Gegenstand der Erfindung ist auch das erfindungsgemäße Verfahren, bei dem die Abtrennung des Analyten mittels einer Vorrichtung mit mindestens einer ersten Kammer 1 und mindestens einer zweiten Kammer 2 erfolgt, wobei in der in das des ersten Kammer 1 eine komplexe Probe 15, die gegebenenfalls in einer Flüssigkeit aufgenommen wurde, angeordnet ist und die Probe 15 mit ferromagnetischen Partikeln 10, an deren Oberfläche der den Analyt bindenden immobilisierte Ligand 20 angeordnet ist, mit einer mittleren Größe von 0,3-30 µm in Kontakt gebracht worden ist,

in der mindestens zweiten Kammer 2 eine flüssige Phase vorliegt und ein Magnetfeld durch einen in die zweite Kammer 2 tauchenden Magneten 3 auf die erste Kammer 1 einwirkt, wodurch die ferromagnetischen Partikel durch eine die erste Kammer 1 und zweite Kammer 2 trennende poröse Trennwand 4, die Poren aufweist, die Partikel mit einer Partikelgröße >100 µm zurück hält, in die zweite Kammer 2 eindringen und danach weiter prozessiert werden.

Die Erfindung wird im Folgenden näher beschrieben.

Gemäß der Erfindung werden solide ferromagnetische Partikel welche eine ferromagnetische Legierung aus Mangan Mangan/ Zink Ferrit enthalten oder daraus bestehen in einem Größenbereich zwischen 0,3 - 50 µm (d99), vorzugsweise solche, deren mittlerer Durchmesser (d50, Gaußverteilung) 6-8 µm beträgt und an deren Oberfläche ein den Analyt bindender Ligand oder eine ionische Gruppe immobilisiert vorliegt. Überraschend wurde gefunden, dass sich ferromagnetische Partikel im Größenbereich von 0,3 - 50 µm sehr gut durch einfaches Schütteln in komplexen Proben dispergieren und nachfolgend durch einfache Magneten vorzugsweise in Form eines einfachen umhüllten Magnetstabes abtrennen und weiterverarbeiten lassen. Überraschend ist weiterhin der Befund, dass die dabei auftretende permanente Magnetisierung die Wasch-, Elutions-, bzw. Extraktions- oder Detektionsschritte nicht wesentlich behindert.

Damit ist das erfindungsgemäße Verfahren auch zum Einsatz sehr komplexer Matrices geeignet. Bei den nach dem erfindungsgemäßen Verfahren zu verwendenden Partikeln behinderte ein "Umhüllungseffekt" von Fetten oder anderen Matrices-Bestandteilen, wie weiter oben als Nachteil bei der Verwendung von paramagnetischen Partikeln und Rohmilch beschrieben, die Abtrennung aus der Probe nicht.

Gegenstand der vorliegenden Erfindung ist auch ein Verfahren, bei dem die Abtrennung des Analyten mittels einer Vorrichtung mit mindestens einer ersten Kammer 1 und mindestens einer zweiten Kammer 2 erfolgt, wobei in der ersten Kammer 1 eine komplexe Probe 15, die gegebenenfalls in einer Flüssigkeit aufgenommen wurde, angeordnet ist und die Probe 15 mit ferromagnetischen Partikeln 10 welche eine ferromagnetische Legierung aus Mangan/ Zink Ferrit enthalten oder daraus bestehen, an deren Oberfläche der den Analyt bindenden immobilisierte Ligand 20 angeordnet ist, mit einer mittleren Größe von 0,3-50 µm, insbesondere in Kontakt gebracht worden ist, in der mindestens zweiten Kammer 2 eine flüssige Phase vorliegt und ein Magnetfeld durch einen in die zweite Kammer 2 tauchenden Magneten 3, insbesondere ein an einem Stab befestigten Magneten, auf die erste Kammer 1 einwirkt, wodurch die ferromagnetischen Partikel 10 durch eine die erste Kammer 1 und zweite Kammer 2 trennende poröse Trennwand 4, die Poren von > 100 µm aufweist, in die zweite Kammer 2 eindringen und danach weiter prozessiert werden. An den Magneten 3 gebundene ferromagnetischen Partikel 10 können insbesondere durch Abstreifen abgelöst werden. Die beschriebene Vorrichtung ist mithin zur Verwendung im erfindungsgemäßen Verfahren geeignet.

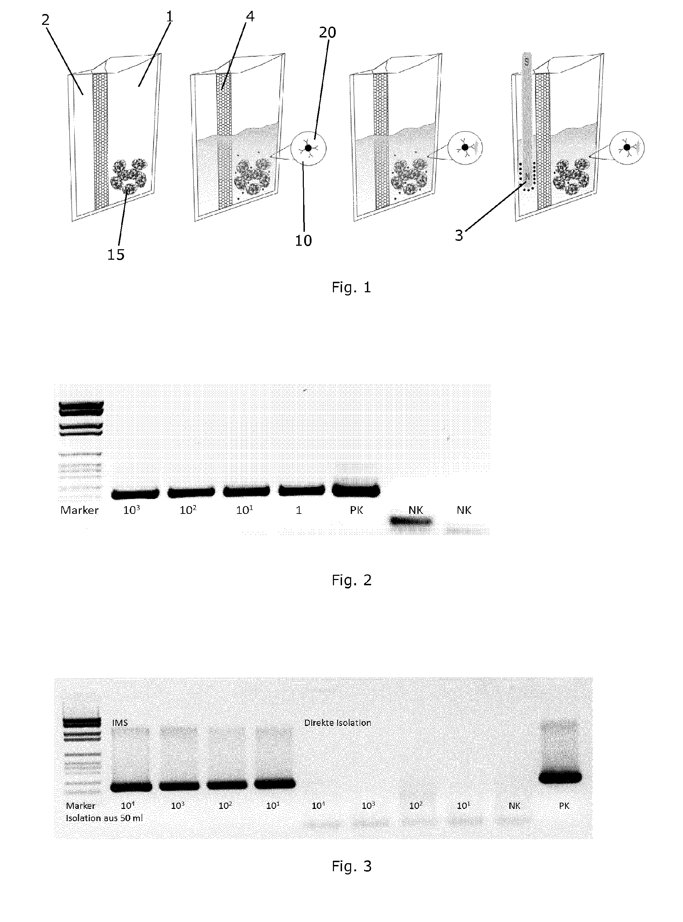

Besonders günstig hat sich dabei eine Anordnung auf Basis eines kommerziell erhältlichen Stomacherbeutels mit integriertem Filtervlies, der den Beutelinhalt in zwei Kammern trennt, erwiesen. Bei diesem in der Lebensmittelmikrobiologie weit eingesetzten Beutel wird die dispergierte Probe 25 g in einer Kammer mit 250 ml flüssigen Medium versetzt und inkubiert. Die Entnahme einer Probelösung erfolgt dann aus der zweiten Kammer, wobei das eingebaute Filtervlies grobe Probenpartikel > 100 µm zurückhält. Überraschend wurde gefunden, dass nach Zugabe der erfindungsgemäßen Partikel in die Probenkammer durch Eintauchen eines Magnets in die Entnahmekammer eine fast vollständige Rückgewinnung der beladenen Magnetpartikel realisiert werden kann. Der Vorgang ist in Fig. 1 skizziert.

Die erfindungsgemäßen ferromagnetischen Partikel sind einfach herstellbar indem industriell gefertigte ferromagnetische Pulver in einem Rührkessel dispergiert und mit einem Liganden, gegebenenfalls über eine in einem vorgelagerten Schritt eingebrachten Zwischenschicht, beschichtet werden. Der Einschluss von nanostrukturierten paramagnetischen Subpartikeln in eine äußere Matrix ist nicht notwendig. Damit wird es möglich kostengünstig große Mengen ferromagnetischer Partikel herzustellen und mit entsprechend hoher Partikeldichte zur Extraktion großer Volumina einzusetzen und damit Nachweisgrenzen zu erreichen, die um mehrere Größenordnungen besser sein können als bei kleinvolumigen Ansätzen. Dieser Vorteil kann insbesondere bei Nachweis von Mikroorganismen auch dazu genutzt werden notwendige Vorinkubationszeiten vor einer PCR deutlich zu verkürzen.

So gelingt zum Beispiel der Nachweis von *Salmonella enterica* subsp. *enterica* nach dem erfindungsgemäßen Verfahren dadurch, dass man zu einer konventionell hergestellten Probe von 25 g Lebensmittel (z.B. Hackfleisch) in 250 ml Peptonwasser nach einer kurzen Vorinkubation von typischerweise 4 h - 7 h in den Stomacher-Beutel zusätzlich 500 µl einer Dispersion (1 g/ml) von ferromagnetischen Partikeln (d= 8µm, MnZn Ferrit), die über eine Zwischenschicht bestehend aus Aminozellulosecarbamat mit 1 mg pro Gramm Partikel anti-Salmonella Antikörper beschichtet wurden, gibt.

Nach einer 1 stündigen Inkubation bei 37 C, unter ständiger, sanfter Bewegung der Probe (Schüttelinkubator), in der sich die Magnetpartikel mit dem ggf. in der Probe befindlichen Analyten (z. B. *Salmonella enterica* subsp.) verbinden, kann die Separation unter Zuhilfenahme eines Magneten (z. B. Neodym-Magneten mit ca. 4 kg Haftkraft) erfolgen. Dazu wird ein, mit einem Plastik-Einweg-Röhrchen umhüllter Magnet, welcher sich zweckmäßigerweise an einem Stab befindet, hinter das Vlies des Stomacherbeutels eingeführt. Dies hat den Effekt, dass zwar die Magnetpartikel mit dem ggf. gebundenen Target das Vlies des Filterbeutels passieren können, grobe Bestandteile der Probe, insbesondere der Lebensmittelprobe aber zurückgehalten werden. Dabei kann das Vlies sowohl aus papierartigem Vlies bestehen und seitlich angeordnet sein, als auch ganzseitig angebracht sein und aus kunststoffartigem Vlies bestehen.

Die Magnetpartikel werden durch das Vlies hindurch zum Magneten gezogen.

Nach wenigen Minuten ist der überwiegende Teil der Magnetpartikel gebunden und der Magnet kann zusammen mit den gebundenen Partikeln entfernt werden.

Zum Waschen der Partikel wird der Magnet, der sich in einer Umhüllung befinden kann, in ein Gefäß, z. B. mit 50 ml Fassungsvermögen, gegeben, in dem sich eine geeignete Menge Waschpuffer (z.B. 5 ml PBS-Puffer) befindet. Nach der Entfernung des Magnets verbleiben die Partikel im Puffer. Der Magnet wird aus der Umhüllung entfernt und kann nun von außen an das Gefäß gehalten werden, um den Waschpuffer durch einfaches Ausgießen zu entfernen, die Partikel werden dabei durch den an der Außenwand des Gefäßes befindlichen Magneten an der Innenwand des Gefäßes festgehalten. Nach einen zweiten Waschschritt zum Beispiel mit 1 ml PBS-Puffer können die an den Partikeln gebundenen Analyten, z. B. Salmonellen entweder für eine DNA-Extraktion vor einer PCR mittels an sich bekannter Verfahren und Arbeitsmittel, z. B. einem kommerziell erhältlichen Aufreinigungskit, weiter bearbeitet werden, wobei die Partikel vor dem Auftrag auf die Silica-Säule einfach entfernt werden können. Die an den Partikeln gebundenen Analyten, z. B. Salmonellen, können auch durch einen einfachen Hitzeschritt lysiert (beispielsweise im Fall von Salmonellennachweis 100 °C/10 min) und der DNA-haltige Überstand direkt in die PCR eingesetzt werden.

Mit diesem Verfahren lassen sich sicher Nacheisgrenzen erreichen, die Grenzwerten von 1CFU/25 g Hackfleisch entsprechen.

Die Erfindung wird an Hand der folgenden Ausführungsbeispiele näher erläutert.

### Ausführungsbeispiel 1:

Herstellung von ferromagnetischen anti-Salmonellenpartikeln mit quervernetztem BSA

### Material:

Albumin Fraktion V (BSA), biotinfrei (ROTH; Art.No. 0163.2)
MnZn-Ferrit-Pulver (MnZnP, d10 2µm, d50 8µm, d99 30µm aufgemahlen, Tridelta, Art. Nr. 2077)
PBS-Puffer pH 7,4 0,3 M (8,596 g/l Na₂HPO₄*12H₂O; 0,828 g/l NaH₂PO₄*H₂O,7,02 g/l NaCl)
Lagerpuffer: 0,2 % BSA in PBS; pH 7,4)
Glutardialdehyd (25%) (Merck; 8.14393.0100)
Monoklonaler Anti-Salmonella Antikörper (Sifin GmbH, Clon 09)

### Durchführung:

1 g BSA werden in einem Becherglas auf einem Magnetrührer in 50 ml PhosphatPuffer gelöst. Anschließend werden 5 g MnZn-Ferrit-Partikel zugegeben und der Ansatz wird bei RT über Nacht gerührt. Am nächsten Tag wird der Ansatz in zwei 50 ml Zentrifugenröhrchen aufgeteilt 2 x 25 ml) und 2 x 10 min mit einer Ultraschallsonotrode (20 W) beschallt, mit einem Dauermagneten abgetrennt und drei Mal mit 50 ml PBS Puffer gewaschen.

100 mg der Partikel werden in 1 ml einer 2% igen Glutardialdehydlösung in PBS aufgenommen und 3 h bei RT geschüttelt. Anschließend wird mit 3 x 5 ml PBS gewaschen waschen und 1 mg Antikörper in 1 ml PBS zugegeben. Die Dispersion wird unter Schütteln über Nacht bei 4°C inkubiert und am nächsten Tag mit 3x5 ml PBS gewaschen. Anschließend werden die Partikel in 1 ml Lagerpuffer aufgenommen (10 %ige Dispersion).

### Ausführungsbeispiel 2:

Herstellung ferromagnetischer anti-Salmonella-Partikel mit Aminocellulosecarbamat-Zwischenschicht via Bernsteinsäureanhydrid/EDC/sulfo-NHS

### Material

MnZn-Ferrit-Pulver (MnZnP, d10 2µm, d50 8µm, d99 30µm aufgemahlen, Tridelta, Art. Nr. 2077)
Ethylendiamin-Carbamat-Cellulose (Mirna Ltd. Madrid Spanien) Bernsteinsäureanhydrid (Merck; 8.00638.1000)
Pyridin (Merck; 1.07463.0500)
EDC (1-Ethyl-3-[3-dimethylaminopropyl]carbodiimid HCl) (AppliChem; A1438,0025)
sulfo-NHS (sulfo-N-Hydroxysuccinimid) (Shanghai Medpep; 40106)
mAk anti-Salmonella (Sifin GmbH, Clon 09; 1,4 mg/ml in PBS-Puffer) N,N-Dimethylformamid
Reinstwasser
MES-Puffer (2-(N-Morpholino)ethansulfonsäure-Puffer; 0,1 M; pH 5,5) (Roth; 4256.3)
PBS-Puffer analog Ausführungsbeispiel 1
Lagerpuffer-analog Ausführungsbeispiel 1
Bradford Reagenz (125 mg Coomassie-Brillant-Blau G250 (AppliChem; A3480,0025); 60 ml Ethanol (VWR; 20821.321), 125 ml Phosphorsäure 85 % (Aldrich; 21,510-4))

### Durchführung

1,0 g MnZn-Ferrit-Partikel werden in ein 2-ml-Eppendorftube eingewogen und für 15 min mit 1ml frisch gelöster 0,1%iger EDA-Carbamat-Cellulose-Lösung in DMF im Überkopfschüttler behandelt. Anschließend werden die Partikel fünf Mal mit je 1 ml DMF gewaschen.

Die modifizierten Partikel werden in ein neues 2-ml-Eppendorftube überführt. Nach Zugabe von 1 ml 0,5M Lösung von Bernsteinsäureanhydrid in DMF und 5µl Pyridin werden die Partikel über Nacht im Überkopfschüttler geschüttelt. Am nächsten Morgen wird vier Mal mit Wasser und zwei Mal mit MES-Puffer gewaschen. Danach werden die Partikel für 15 min in 0,5 ml 0,10 M Lösung von EDC in MES-Puffer geschüttelt bevor nach Zugabe von 0,5 ml 0,25 M Lösung Sulfo-NHS in MES-Puffer für weitere 30 min geschüttelt wird. Nach dem Waschen der Partikel mit je 1 ml MES- sowie PBS-Puffer werden diese mit 1 ml Antikörperlösung (1,4 mg/ml in PBS-Puffer) versetzt und für 2h im Überkopfschüttler inkubiert. Anschließend wird mit 1 ml PBS-Puffer gewaschen. Die Lagerung der Partikel erfolgt in 1 ml Lagerpuffer.

Die Antikörperlösung nach dem Kopplungsprozess sowie die Waschlösung werden per Bradford-Assay in an sich bekannter Weise auf das Vorhandensein von ggf. ungebundenen Antikörpern untersucht. Es konnten nur noch geringe Antikörpermengen nachgewiesen werden, d.h. es wurden 1,35 mg Antikörper an 1 g MnZn-Ferrit-Partikel gekoppelt.

### Ausführungsbeispiel 3:

Herstellung von paramagnetischen Partikeln für Vergleichszwecke

### Material:

SiMAG-Carboxyl Paramagnetische Partikel (chemicell GmbH, 1402-1) Wasch- und Kopplungspuffer:
0.1 M 2-(N-Morpholino)ethanesulfonic acid (MES), pH 5.0
MES-Puffer (2-(N-Morpholino)ethansulfonsäure-Puffer; 0,1 M; pH 5,0 (Roth; 4256.3)
EDC (1-Ethyl-3-[3-dimethylaminopropyl]carbodiimid HCl) (AppliChem; A1438,0025)
Block- und Lagerpuffer: PBS, 0,1 % BSA, 0,05 % Natriumazid; pH 7,4 PBS-Puffer pH 7,4 0,3 M (8,596 g/l Na₂HPO₄*12H₂O; 0,828 g/l NaH₂PO₄*H₂O,7,02 g/l NaCl)
Monoklonaler Anti-Salmonella Antikörper (Sifin GmbH, Clon 09)

### Durchführung

Es werden 10 mg SiMAG-Carboxyl Partikel zweimal mit 1 ml MES-Puffer gewaschen. Zwischen jedem Waschschritt werden die Partikel mit einem Magneten zurückgehalten. Nach dem zweiten Waschritt werden die Partikel in 0,25 ml MES-Puffer resuspendiert, welcher 10 mg EDC enthält. Nach einer 10 minütigen Inkubation unter Schütteln bei Raumtemperatur werden 50 µg monoklonaler Anti-Salmonella Antikörper zugegeben und für weitere 2 h unter Schütteln bei Raumtemperatur inkubiert. Nach dreimaligem Waschen der Partikel mit je 1 ml PBS-Puffer werden die Partikel in Block- und Lagerpuffer resuspendiert und bis zur Verwendung bei 4 °C gelagert.

### Ausführungsbeispiel 4:

Extraktion von Salmonella aus einer Hackfleischprobe mit ferromagnetischen Partikeln

### Materialien:

MnZn-Ferrit-Partikel, hergestellt wie in Ausführungsbeispiel 2 beschrieben PBS Puffer analog Ausführungsbeispiel 1
Waschpuffer 1: PBS + 0,1% BSA, sterilfiltriert
Waschpuffer 2: PBS + 0,05% Tween 20
gepuffertes Peptonwasser (Oxoid, CM1049)
Rappaport-Vassiliadis-Sojamehlpepton-Bouillon (Oxoid, CM0866)
Müller-Kauffmann mit Novobiocin (Oxoid, CM1048)
sterile Probenbeutel für Stomacher 400 (Meintrup dws Laborgeräte, ME001004, Typ M mit ganzseitigem Filter)
sterile Probenbeutel für Stomacher 400 (Meintrup dws Laborgeräte, ME001006a, Typ P mit Filterstreifen zum Pipettieren)
15 ml Röhrchen (Greiner Bio One, Art.-Nr.: 188271)
50 ml Röhrchen (Greiner Bio One, Art.-Nr.: 227261)
Stabmagnet Ø 10 mm, Höhe 40 mm (Webcraft GmbH, S-10-40-N)
Magnetstab-Entferner (Roth, E449.1)

### Durchführung

Nachweis von *Salmonella enterica* subsp. aus Lebensmittelproben

### a) klassische Voranreicherungsmethode für Vergleichsuntersuchungen

Die Untersuchung erfolgt nach den Vorschriften der Referenzmethode nach ASUV § 64 LFGB. (Referenzverfahren zum horizontalen Nachweis von Salmonellen in Lebensmitteln und Futtermitteln gemäß L 00.00-20, ASUV § 64 LFGB (entspricht DIN EN ISO 6579:2003))

25 g Hackfleisch werden zusammen mit 250 ml gepuffertem Peptonwasser in einen sterilen Probenbeutel, sog. Stomacher-Beutel gegeben, und für 24 h bei 37°C bebrütet (Voranreicherung). Nachfolgend werden 0,1 ml der Voranreicherung in 10 ml Rappaport-Vassiliadis-Sojamehlpepton-Bouillon (RVS) und 1 ml der Voranreicherung in 10 ml Müller-Kauffmann mit Novobiocin (MKTTn) überführt und für weitere 24 h bei 41,5 °C (Rappaport-Vassiliadis-Sojamehlpepton-Bouillon) bzw. bei 37 °C (Müller-Kauffmann mit Novobiocin) bebrütet. Nach 24 Stunden Inkubation erfolgt eine Subkultivierung auf dem festen Referenzselektivnährboden Xylose-Lysin-Desoxycholat-Agar (XLD) und einem zweiten Medium nach Wahl. Nach einer weiteren 18 bis 27-stündigen Inkubation werden die Reinkulturen bestätigt.

### b) Voranreicherung mit integrierter Extraktion von Salmonella auf Basis von ferromagnetischen Partikeln

25 g Hackfleisch werden zusammen mit 250 ml gepuffertem Peptonwasser in einen sterilen Probenbeutel, sog. Stomacher-Beutel gegeben, und für 4 - 7 h bei 37 °C bebrütet (Voranreicherung).

Vor dem Einsatz für die Magnetseparation werden die Partikel entsprechend gewaschen, um das Konservierungsmittel zu entfernen. Dazu werden zu 500 µl der Partikeldispersion (1 g/ml) 1 ml Waschpuffer gegeben und die Pufferphase durch Ausgießen entfernt (die Partikel werden dabei durch einen an die Außenwand des Reaktionsgefäßes gehaltenen Magneten zurückgehalten), anschließend wird der Waschschritt mit Waschpuffer 2 wiederholt und die Partikel in 1 ml Waschpuffer 2 aufgenommen.

Diese Dispersion wird mit der vorinkubierten Probe vereinigt und 1 Stunde bei 37°C unter ständiger, sanfter Bewegung (Schüttelinkubator) inkubiert. Die Separation erfolgt unter Zuhilfenahme eines starken Neodym-Magneten (4 kg Haftkraft). Dazu wird ein mit einem 15 ml Röhrchen umhüllter Magnet, welcher sich an einem Magnetstab-Entferner befindet, hinter das Vlies des Stomacher-Beutels eingeführt. Dies hat den Effekt, dass zwar die Magnetpartikel mit dem ggf. gebundenen Target das Vlies des Filterbeutels passieren können, grobe Bestandteile der Lebensmittelprobe aber zurückgehalten werden. Dabei kann das Vlies sowohl aus papierartigem Vlies (ME001006a) bestehen und seitlich angeordnet sein, als auch ganzseitig angebracht sein und aus kunststoffartigem Vlies (ME001004) bestehen.

Nach wenigen Minuten sind die Magnetpartikel an der Außenseite des Röhrchens durch den im Röhrchen befindlichen Magneten gebunden können aus der Kammer des Stomacher-Beutels gezogen werden. Die am Röhrchen durch den im Röhrchen befindlichen Magneten gebundenen Magnetpartikel werden in ein 50 ml Röhrchen, das 5 ml PBS-Puffer enthält gegeben. Nach dem ruckartigen Herausziehen des Magnets aus dem 15 ml Röhrchen verbleiben die Partikel im Puffer. Das leere 15 ml Röhrchen wird entfernt, die Partikel befinden sich nun im PBS-Puffer in einem 50 ml Röhrchen. Der Magnet wird nun von außen an das 50 ml Röhrchen gehalten werden, wobei die Magnetpartikel an der Wand fixiert werden um den Waschpuffer durch einfaches Ausgießen zu entfernen zu können. Es erfolgt ein zweiter Waschschritt mit 1ml PBS-Puffer.

Danach können die an den Partikeln gebundenen Salmonellen derart ausgeschlossen werden (z. b. durch eine chemische oder thermische Lyse in an sich bekannter Weise) das entweder eine direkte PCR ohne weitere DNA-Extraktion oder eine DNA-Extraktion mittels eines geeigneten Aufreinigungsverfahrens vor der PCR durchgeführt werden kann.

### Ergebnis

Anhand von künstlich kontaminierten Proben konnte gezeigt werden, dass bei einer siebenstündigen Vorinkubation der Lebensmittelproben ein Nachweis bis zu 1 KBE/250 ml Probe möglich ist.
Fig. 2: DNA-Isolation mit kommerziellem Kit (Macherey-Nagel NucleoSpin Food Kit, 740945.250).
   PCR als nested PCR (Primer erste PCR: invA-1486F:
   ACAAAACATATGCTGGACCAA; invA-1875R:
   CTTAAGTGTAATGAGATCCATCAAAT; 390 bp Produkt. Primer zweite PCR: InvA-1597F: GAACGTGTTTCCGTGCGTAA; InvA-1855R:
   TCAAATTAGCGGAGGCTTCC; 259 bp Produkt.)

### Ausführungsbeispiel 5:

Anwendung der ferromagnetischen Partikel zum Nachweis von *Salmonella enterica* subsp. *enterica* aus Kulturmedium > 50 ml Volumen.

### Material

MnZn-Ferrit-Partikel, hergestellt wie in Ausführungsbeispiel 1 beschrieben
PBS Puffer analog Ausführungsbeispiel 1
Waschpuffer 1: PBS + 0,1% BSA, sterilfiltriert
Waschpuffer 2: PBS + 0,05% Tween 20
gepuffertes Peptonwasser (Oxoid, CM1049)

Stabmagnet Ø 10 mm, Höhe 40 mm (Webcraft GmbH, S-10-40-N)

### Durchführung

Vor dem Einsatz für die Magnetseparation werden die Partikel entsprechend gewaschen, um das Konservierungsmittel zu entfernen. Dazu werden zu 500 µl der Partikeldispersion (1 g/ml) 1 ml Waschpuffer gegeben und die Pufferphase durch Ausgießen entfernt (die Partikel werden dabei durch einen an die Außenwand des Reaktionsgefäßes gehaltenen Magneten zurückgehalten), anschließend wird der Waschschritt mit Waschpuffer 2 wiederholt und die Partikel in 1 ml Waschpuffer 2 aufgenommen.

Eine über-Nacht-Kultur von *Salmonella enterica* subsp. *enterica* wird in an sich bekannter Weise quantifiziert und logarithmisch verdünnt. Verdünnungsstufen von 1 - 10000 KBE/ml werden 50 - 250 ml frischem, unbebrütetem Kulturmedium (z. B. gepuffertes Peptonwasser) zugeführt.

Nach einer Vorinkubationszeit von 3 - 4 Stunden bei 37 °C werden die gewaschenen Partikel zugefügt (100 µl Partikel in 50 ml Medium) und für 1 h bei 37°C im über-Kopf-Schüttler bei 15 rpm inkubiert. Nach Ablauf der Inkubationszeit werden die Partikel mit einem Stabmagneten an der Innenwand des Kulturgefäßes fixiert und der Überstand ausgegossen. Die Partikel werden mit 2 x 1 ml PBS-Puffer gewaschen. Danach können die an den Partikeln gebundenen Salmonellen derart ausgeschlossen werden (z. b. durch eine chemische oder thermische Lyse in an sich bekannter Weise) das entweder eine direkte PCR ohne weitere DNA-Extraktion oder eine DNA-Extraktion mittels eines geeigneten Aufreinigungsverfahrens vor der PCR durchgeführt werde kann.

Fig 3.: Aus 50 ml mit einer definierten Menge an Salmonellen versetztem Kulturmedium konnte nach 1 h Vorinkubation gezeigt werden, das die Anwendung der ferromagnetischen Partikel bis zu 10 KBE/50 ml positiv ist. Ein direkter Nachweis von standardmäßig 1 ml war nicht möglich.

DNA-Isolation mit kommerziellem Kit (Macherey-Nagel NucleoSpin Food Kit, 740945.250).
PCR als nested PCR (Primer erste PCR: invA-1486F:
ACAAAACATATGCTGGACCAA; invA-1875R: CTTAAGTGTAATGAGATCCATCAAAT; 390 bp Produkt. Primer zweite PCR: InvA-1597F: GAACGTGTTTCCGTGCGTAA; InvA-1855R: TCAAATTAGCGGAGGCTTCC; 259 bp Produkt.)

### Ausführungsbeispiel 6:

Anwendung von ferromagnetischen Partikeln in künstlich kontaminierter Rohmilch im Vergleich zu paramagnetischen Partikeln

### Material

MnZn-Ferrit-Partikel, hergestellt wie in Ausführungsbeispiel 2 beschrieben Paramagnetische Partikel, hergestellt wie in Ausführungsbeispiel 3 beschrieben 50 ml Röhrchen (Greiner Bio One, Art.-Nr.: 227261)
Stabmagnet Ø 10 mm, Höhe 40 mm (Webcraft GmbH, S-10-40-N)

### Durchführung

### a) Anwendung von paramagnetischen Partikeln in Rohmilch

Es wurden 5 mg Partikel, dispergiert in 500 µl Block- und Lagerpuffer (Siehe Anwendungsbeispiel 3) wie in Ausführungsbeispiel 4 beschrieben gewaschen und zu 50 ml Rohmilch zugegeben und durch sanftes Schütteln für 30 min bei Raumtemperatur inkubiert.
Durch Fette und unlösliche Bestandteile der Rohmilch wurden die paramagnetischen Partikeln förmlich "umschlossen" und konnten mit einem Magneten nicht zurückgewonnen werden. Zumeist waren keine paramagnetischen Partikel mehr sichtbar. Eine Zentrifugation (500 x g für 2 min) zur Aufkonzentrierung der Partikel am Boden des Gefäßes bewirkte, dass die Rohmilch eine Sahneschicht ausgebildet hat, auf deren Oberfläche die paramagnetischen Partikel saßen. So eingeschlossen waren sie nicht mehr rückzugewinnen, bzw. teilweise auch nicht mehr sichtbar.

### b) Anwendung von ferromagnetischen Partikeln in Rohmilch

Ferromagnetische Partikel heben sich sehr deutlich in der Rohmilch ab, da sie nicht wie paramagnetische Partikel hellbraun gefärbt sind, sondern eine tiefschwarze Farbe aufweisen. Die natürliche Auftrennung der Rohmilch in Sahne- und Molkefraktion behinderte in keiner Weise die Rückgewinnung der ferromagnetischen Partikel. Die ferromagnetischen Partikel sanken teilweise zu Boden und wurden nicht in der Sahnefraktion festgehalten. Wenn ferromagnetische Partikel von Fettbestandteilen der Milch umschlossen waren, konnten diese auf Grund der starken magnetischen Kräfte dennoch an der Innenwand des Gefäßes festgehalten werden.

### Ausführungsbeispiel 7:

Extraktion mit paramagnetischen Partikeln mit anti-Salmonella Beschichtung zum Nachweis von Salmonellen in einer Lebensmittelprobe z.B. Hackfleisch-Probe

### Material

Paramagnetische Partikel hergestellt wie in Ausführungsbeispiel 3 beschrieben

Storage Buffer: PBS, 0.1 % BSA, 0.05 % Natriumazid

sterile Probenbeutel für Stomacher 400 (Meintrup dws Laborgeräte, ME001004, Typ M mit ganzseitigem Filter)

sterile Probenbeutel für Stomacher 400 (Meintrup dws Laborgeräte, ME001006a, Typ P mit Filterstreifen zum Pipettieren)
gepuffertes Peptonwasser (Oxoid, CM1049)
15 ml Röhrchen (Greiner Bio One, Art.-Nr.: 188271)
50 ml Röhrchen (Greiner Bio One, Art.-Nr.: 227261)

Stabmagnet Ø 10 mm, Höhe 40 mm (Webcraft GmbH, S-10-40-N)

Magnetstab-Entferner (Roth, E449.1)

Es wurden 5 mg Partikel, dispergiert in 500 µl Block- und Lagerpuffer (Siehe Anwendungsbeispiel 3) wie in Ausführungsbeispiel 4 beschrieben gewaschen.

Eine über-Nacht-Kultur von *Salmonella enterica* subsp. *enterica* wird in an sich bekannter Weise quantifiziert und logarithmisch verdünnt. Verdünnungsstufen von 1 - 10000 KBE/ml werden 250 ml gepuffertem Peptonwasser zugeführt. 25 g Hackfleisch werden zusammen mit 250 ml künstlich kontaminiertem gepuffertem Peptonwasser in einen sterile Probenbeutel, sog. Stomacher-Beutel gegeben, und für 4 - 7 h bei 37 °C bebrütet (Voranreicherung).

Diese gewaschene Magnetpartikel-Dispersion wird mit der vorinkubierten Probe vereinigt und 1 Stunde bei 37°C unter ständiger, sanfter Bewegung (Schüttelinkubator) inkubiert. Die Separation erfolgt unter Zuhilfenahme eines starken Neodym-Magneten (4 kg Haftkraft). Dazu wird ein mit einem 15 ml Röhrchen umhüllter Magnet, welcher sich an einem Magnetstab-Entferner befindet, hinter das Vlies des Stomacher-Beutels eingeführt. Dies hat den Effekt, dass zwar die Magnetpartikel mit dem ggf. gebundenen Target das Vlies des Filterbeutels passieren können, grobe Bestandteile der Lebensmittelprobe aber zurückgehalten werden. Dabei kann das Vlies sowohl aus papierartigem Vlies (ME001006a) bestehen und seitlich angeordnet sein, als auch ganzseitig angebracht sein und aus kunststoffartigem Vlies (ME001004) bestehen.

Die paramagnetische Partikel sind in der Probe kaum sichtbar und lassen sich nur sehr schwer von Fett und groben Bestandteilen der Probe zu trennen. Nachdem sich der Magnet eine längere Zeit (> 5 min) hinter dem Stützvlies befand (bei ferromagnetischen Partikeln reichen 1 - 2 Minuten) blieb eine geringe Menge paramagnetische Partikel am Magneten hängen.

Wie in Ausführungsbeispiel 4 beschrieben werden die paramagnetische Partikel gewaschen, die ggf. gebundenen Salmonellen aufgeschlossen und einer geeigneten PCR-Reaktion zugeführt. Ein positiver Nachweis bei einer künstlichen Kontamination mit 1000 KBE pro Probe war nicht möglich.

### Ausführungsbeispiel 8:

Sandwichassay mit ferromagnetischen Partikeln mit anti-Salmonella Beschichtung wie in Ausführungsbeispiel 2 beschrieben und löslichen Farbpartikelkonjugaten

### Material

MnZn-Ferrit-Partikel (8 +/-2 µm) beschichtet mit anti-Salmonella entsprechend Ausführungsbeispiel 2
Probenpuffer (PP): 1 Teil 5,5% Casein Buffer Concentrate (CBC) #CBC1, Fa. SDT, 3 Teile PBS pH 7,4
Waschpuffer (WP): PBS pH 7,4 + 0,05% Tween20
Blockpuffer (BP): WP + 5% Magermilch

| | |
|---|---|
| Probe: | Hackfleischprobe versetzt mit *Salmonella enterica* subsp. *enterica* |
| Farbpartikelkonjugat: | Anti-Salmonella- ABICAP Rot (Senova GmbH Jena), 1mg/ml |
| Elutionslösung : | Ethanol 96% |
| Probenextraktion: | |

Es wird verfahren wie im Ausführungsbeispiel 4 beschrieben.

### Nachweisreaktion

Die Magnetpartikel werden in einem 2 ml Reaktionsgefäßen mit 1 ml PBS dispergiert. Anschließend werden 500 µl Farbpartikelkonjugat zugegeben und es wird 10 min bei Raumtemperatur auf einem Schüttler inkubiert. Anschließend wird das Reaktionsgefäß in eine Magnetständer gegeben, der Überstand wird dekantiert und es wird drei Mal mit 1,50 ml PBS Puffer gewaschen. Anschließend werden die gebundenen Farbpartikel durch Zugabe von 1 ml Ethanol aufgelöst, die Lösung wird abgetrennt und die optische Dichte (OD) wird mit einem Spektralphotometer bei einer Wellenlänge von 525 nm und einer Schichtdicke von 1 cm bestimmt.

### Ergebnisse:

| Keimzahl/ml | OD (525 nm) |
|---|---|
| 0 | 0,101 |
| 10 | 0,125 |
| 100 | 0,142 |
| 1000 | 0,378 |
| 10.000 | 0,986 |
| 100.000 | 1,478 |
| 1.000.000 | 1,855 |

### Ausführungsbeispiel 9

Verwendung von monoklonalen Anti-Adenovirushexon-Antikörper-gekoppelten Magnetpartikeln zum Nachweis von Adenoviren in Gewebeproben

### Materialien:

MnZn-Ferrit-Partikel, hergestellt wie in Ausführungsbeispiel 1 beschrieben PBS Puffer analog Ausführungsbeispiel 1
Lysis Matrix H Zirkonium Lysiskugeln (MP Biomedicals Germany, 116917050) FastPrepInstrument (MP Biomedicals Germany, 116004500)
15 ml Röhrchen (Greiner Bio One,Art.-Nr.: 188271)

Stabmagnet Ø 10 mm, Höhe 40 mm (Webcraft GmbH, S-10-40-N)

NucleoSpin Tissue Kit (Macherey-Nagel, 740952)

### Durchführung:

Ein Gramm Lymphknotengewebe wurde mit 9 ml PBS versetzt und mittels Zirkonium Lysiskugeln im FastPrep Instrument homogenisiert (3x 30 Sekunden mit 6,5m/s). Der 10%ige Gewebebrei wurde mit 1x10⁴ infektiösen Einheiten Adenovirus 5 versetzt und 30 Sekunden auf einem Laborschüttler in einem 15 ml Röhrchen gemischt. Dem Gemisch wurden 200µl PBS-gewaschene ferromagnetische Partikel zugesetzt. Dies entspricht 20µl einer 1 Gramm pro 1 ml Stamm-Partikel Lösung. Das Waschen der Partikel erfolgte analog zu Ausführungsbeispiel 4.

Das Gemisch wurde 1 h bei 37°C in einem drehenden und wippenden Inkubator gemischt, wobei die Magnetpartikel ständig im Gewebebrei bewegt wurden und keine sichtbare Sedimentation stattfand.

Die ferromagnetische Partikel wurden anschließend mittels des Permanentmagneten von außen an die Wand des Röhrchens gezogen und der Gewebebrei wurde abpipettiert. Das Röhrchen wurde mit 10 ml PBS aufgefüllt und 30 Sekunden auf einem Laborschüttler geschüttelt, um Gewebereste, die zwischen den ferromagnetische Partikel unspezifisch angelagert waren zu entfernen (Waschschritt). Anschließend wurden die ferromagnetische Partikel wieder an die Röhrchenwand gezogen und der Waschpuffer abpipettiert.

Die ferromagnetische Partikel wurden in 700 µl Lysispuffer (NucleoSpinTissue Kit) für 1h bei 55°C inkubiert und die darin enthaltene Proteinase-K anschließend durch Erhitzen für 20 Minuten auf 98°C inaktiviert. Aus dem Lysat wurde die Nukleinsäure durch Phenolextraktion in bekannter Weise abgetrennt und anschließend die DNA aus der wässrigen Phase mit dem NucleoSpinTissue Kit nach Anleitung des Herstellers gereinigt und in 50 µl H₂O eluiert.

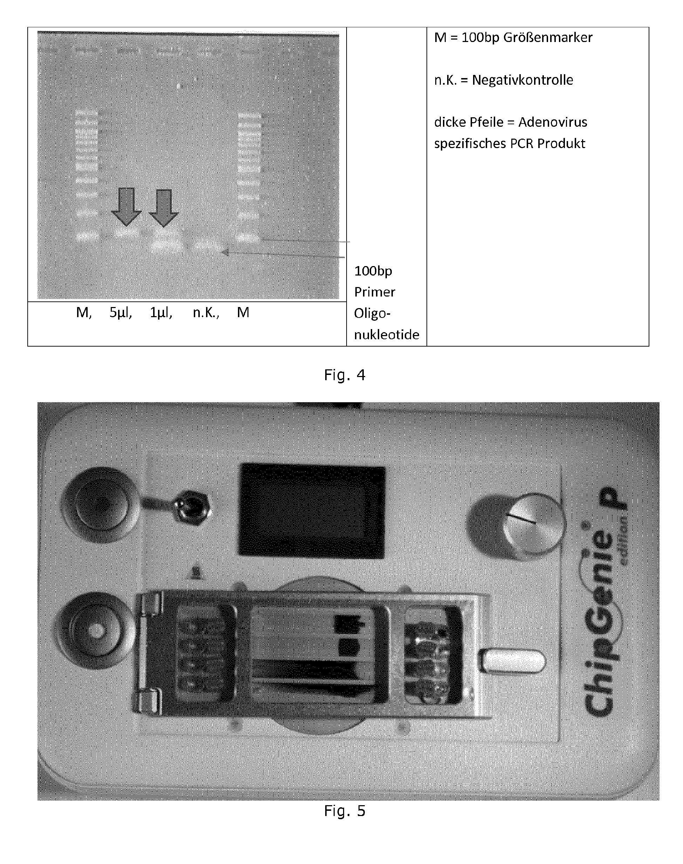

In eine Adenovirus spezifische PCR wurden 5 bzw. 1 µl der eluierten DNA als Template eingesetzt. 1 µl Template reichten aus, um ein spezifisches Produkt zu generieren (Fig. 4, 2%iges Agarosegel, Färbung mit Ethidiumbromid). Da 1 µl Template einem 50zigstel des eingesetzten Spikevirus entspricht, kann davon ausgegangen werden, dass der ferromagnetische Partikelbrei, bei einem Detektionslimit der PCR von 10 Targets/Reaktion, mindestens 2000 PCR-Targetäquivalente aus dem Gewebebrei gebunden hatte.

### Ausführungsbeispiel 10:

Beschichtung von ferromagnetischen Partikeln der Firma Spherotech für Vergleichszwecke nach Anleitung des Herstellers (SpheroTechnical Notes #1-Particles Coating Procedures
http://spherotech.com/tech_SpheroTech_Note_1.html)

### Material:

SPHERO Amino Ferro-Magnetic Particles 1 % w/v 4,32 µm (Cat No. AFM-40-10) SPHERO Carboxyl Ferro-Magnetic Particles 1 % w/v 4,47 µm (Cat No. CFM-40-10)

EDC (1-Ethyl-3-[3-dimethylaminopropyl]carbodiimid HCl) (AppliChem; A1438,0025)

Monoklonaler Anti-Salmonella Antikörper (Sifin GmbH, Clon 09)

### Wasch- und Kopplungspuffer:

Nach Anleitung des Herstellers hergestellte Puffer:
IBS Isotonic Buffered Saline
Phosphatpuffer 0,1 M pH 7,4
Natriumacetat-Puffer 0,01 M, pH 5.0.

### Durchführung

Die Partikel werden als 2,5 % Lösung verwendet und eine 1-Schritt EDC-Kopplung nach Anleitung des Herstellers durchgeführt. Dazu wird in einem Reaktionsgefäß 2 ml Phosphatpuffer 0,1 M pH 7,4 (bei SPHERO Amino Ferro-Magnetic Particles) bzw. 2 ml Natriumacetat-Puffer 0,01 M, pH 5.0 (bei Verwendung der SPHERO Carboxyl Ferro-Magnetic Particles) zusammen mit 0,5 mg Anti-Salmonella Antikörper, 2 ml 2,5 % Partikellösung und 5 mg EDC miteinander vermischt. Anschließend wird die Suspension 2 h auf über-Kopf-Schüttler bei Raumtemperatur inkubiert. Der Überstand wird nach Ablauf der Inkubationszeit entfernt und die Partikel zweimal in 4 ml IBS gewaschen. Abschließend werden die Partikel in 2 ml IBS ausgenommen und bei 4 °C bis zur Verwendung gelagert.

### Ausführungsbeispiel 11:

Fig. 5: Vergleich des magnetischen Verhaltens von SPHERO Amino Ferro-Magnetic Particles 1 % w/v 4,32 µm (Cat No. AFM-40-10) und SPHERO Carboxyl Ferro-Magnetic Particles 1 % w/v 4,47 µm (Cat No. CFM-40-10) in wässrigen Lösungen im Vergleich zu erfindungsgemäßen ferromagnetischen Partikeln wie in Ausführungsbeispiel 4 beschrieben.

In der weiteren Beschreibung werden SPHERO Amino Ferro-Magnetic Particles 1 % w/v 4,32 µm (Cat No. AFM-40-10) und SPHERO Carboxyl Ferro-Magnetic Particles 1 % w/v 4,47 µm als SPHERO-Tech Partikel zusammengefasst, da sie sich in wässrigen Lösungen näherungsweise gleich verhalten.

Es konnte gezeigt werden, dass sich die SPHERO-Tech Partikel kaum von einem Magneten anziehen lassen. Sie am Boden eines Reaktionsgefäßes zu fixieren, um Beispielsweise den Überstand auszugießen, ist nicht möglich. Sie lassen sich sehr leicht in Lösung bringen und sinken sehr langsam zu Boden. Das magnetische Verhalten wurde im ChipShop P-Gerät demonstriert. Hierbei läuft ein unterhalb des vier-Kammer-Chips angebrachter Magnet von links nach rechts und zurück.

Die SPHERO-Tech Partikel lassen sich vom Gerät kaum bewegen. Die magnetische Kraft der Partikel ist nicht ausreichend um die Partikel als klare Bande hin- und her zu bewegen. Die SPHERO-Tech Partikel bilden in den unteren beiden Kammern eine diffuse Bande die nur leicht hin- und her läuft. Die in den oberen beiden Kammern befindlichen erfindungsgemäßen Partikel gemäß Ausführungsbeispiel 4 bilden eine diskrete Bande, so dass die magnetischen Eigenschaften der Partikel erhalten bleiben.

Im Magnetständer wird der Unterschied noch deutlicher.

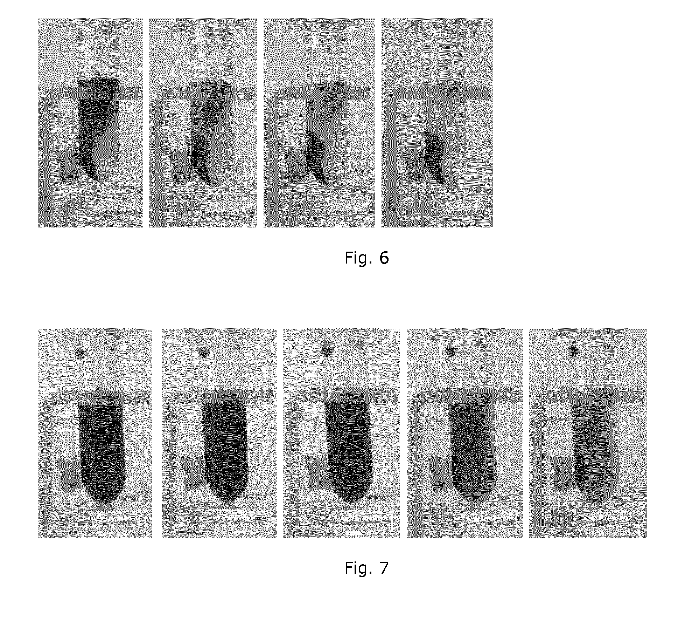

Fig. 6: Ferromagnetische Partikel gemäß Ausführungsbeispiel 4 nach 1 s, 2, s, 3 s und 9 s

Fig. 7: SPHERO-Tech Partikel gemäß Ausführungsbeispiel 11 nach 1 s, 3, s, 9 s, 25 s und 34 s

Die erfindungsgemäßen ferromagnetischen Partikel wie in Ausführungsbeispiel 4 beschrieben, streben sofort zum Magneten und sind innerhalb weniger Sekunden angehaftet. Die SPHERO-Tech Partikel brauchen über 30 Sekunden um sich dort zu sammeln, bei Schwenken des Ständers geht die Bindung sofort verloren. Die erfindungsgemäßen ferromagnetischen MnZn-Ferrit-Partikel nach Ausführungsbeispiel 4 erlauben eine bessere Trennung von dem an den Partikeln anhaftenen Analyten von der umstehenden Lösung.

So zeigen die erfindungsgemäßen ferromagnetischen Partikel nach 9 s eine praktisch vollständige Trennung bzw. Entmischung von der umstehenden Lösung, während die SPHERO-Tech Partikel nach 34 s noch keine vollständige Trennung zeigen. Die Trennwirkung gemessen als Zeit der Trennung/Entmischung der Lösung (Dispersion) der erfindungsgemäßen ferromagnetischen Partikel ist somit etwa um den Faktor 3-4 größer als bei den Vergleichspartikeln mit einem Chromdioxidkern.

### Ausführungsbeispiel 12:

Extraktion mit SPHERO Amino Ferro-Magnetic Particles 1 % w/v 4,32 µm (Cat No. AFM-40-10) und SPHERO Carboxyl Ferro-Magnetic Particles 1 % w/v 4,47 µm (Cat No. CFM-40-10) zum Nachweis von Salmonellen in einer Lebensmittelprobe z.B. Hackfleisch-Probe

### Material

SPHERO Amino Ferro-Magnetic Particles 1 % w/v 4,32 µm (Cat No. AFM-40-10) SPHERO Carboxyl Ferro-Magnetic Particles 1 % w/v 4,47 µm (Cat No. CFM-40-10), gekoppelt mit anti-Salmonella Antikörper wie in Ausführungsbeispiel 10 beschrieben.

sterile Probenbeutel für Stomacher 400 (Meintrup dws Laborgeräte, ME001004, Typ M mit ganzseitigem Filter)

sterile Probenbeutel für Stomacher 400 (Meintrup dws Laborgeräte, ME001006a, Typ P mit Filterstreifen zum Pipettieren)

15 ml Röhrchen (Greiner Bio One, Art.-Nr.: 188271)
50 ml Röhrchen (Greiner Bio One, Art.-Nr.: 227261)
Stabmagnet Ø 10 mm, Höhe 40 mm (Webcraft GmbH, S-10-40-N)
Magnetstab-Entferner (Roth, E449.1)

### Durchführung

Es wurden 500 µl Partikel, hergestellt wie in Ausführungsbeispiel 10 beschrieben, pro Probe eingesetzt.

Eine über-Nacht-Kultur von *Salmonella enterica* subsp. *enterica* wird in an sich bekannter Weise quantifiziert und logarithmisch verdünnt. Verdünnungsstufen von 1 - 10000 KBE/ml werden 250 ml gepuffertem Peptonwasser zugeführt.

25 g Hackfleisch werden zusammen mit 250 ml künstlich kontaminiertem gepuffertem Peptonwasser in einen sterile Probenbeutel, sog. Stomacher-Beutel gegeben, und für 4 - 7 h bei 37 °C bebrütet (Voranreicherung).

Diese gewaschene Magnetpartikel-Dispersion wird mit der vorinkubierten Probe vereinigt und 1 Stunde bei 37°C unter ständiger, sanfter Bewegung (Schüttelinkubator) inkubiert. Die Separation erfolgt unter Zuhilfenahme eines starken Neodym-Magneten (4 kg Haftkraft). Dazu wird ein mit einem 15 ml Röhrchen umhüllter Magnet, welcher sich an einem Magnetstab-Entferner befindet, hinter das Vlies des Stomacher-Beutels eingeführt. Dies hat den Effekt, dass zwar die Magnetpartikel mit dem ggf. gebundenen Target das Vlies des Filterbeutels passieren können, grobe Bestandteile der Lebensmittelprobe aber zurückgehalten werden. Dabei kann das Vlies sowohl aus papierartigem Vlies (ME001006a) bestehen und seitlich angeordnet sein, als auch ganzseitig angebracht sein und aus kunststoffartigem Vlies (ME001004) bestehen.

Die SPHERO Amino Ferro-Magnetic Partikel bzw. SPHERO Carboxyl Ferro-Magnetic Partikel sind in der Probe kaum sichtbar und lassen sich nur sehr schwer von Fett und groben Bestandteilen der Probe zu trennen. Nachdem sich der Magnet eine längere Zeit (> 5 min) hinter dem Stützvlies befand (bei ferromagnetischen Partikeln reichen 1 - 2 Minuten) blieb eine geringe Menge SPHERO Amino Ferro-Magnetic Partikel bzw. SPHERO Carboxyl Ferro-Magnetic Partikel am Magneten hängen.

Wie in Ausführungsbeispiel 4 beschrieben werden die Partikel gewaschen, die ggf. gebundenen Salmonellen aufgeschlossen und einer geeigneten PCR-Reaktion zugeführt. Ein positiver Nachweis bei einer künstlichen Kontamination mit 100 KBE pro Probe war nicht möglich, im Vergleich dazu war der Nachweis von 100 KBE pro Probe bei der Anwendung der Partikel aus Ausführungsbeispiel 4 möglich.

### Referenzliste

1 Mattingly, J.A. (1984): "An enzyme immunoassay for the detection of all Salmonella using a combination of a myeloma protein and a hybridoma antibody". Journal of Immunological Methods 73, S. 147-156.
2 Olsvik, O., Popovic, T., Skjerve, E., Cudjoe, K., Homes, E., Ugelstad, J. , Uhlen, M. (1994): "Magnetic separation techniques in diagnostic microbiology". Clinical Microbiology Reviews 7, S. 43-54.
3 Ugelstad, J., Stenstad, P., Kilaas, L., Prestvik, W.S., Herje, R., Berge, A., Hornes, E. (1993): "Monodisperse magnetic polymer particles. New biochemical and biomedical applications". Blood Purification 11 (6), S. 349-369.
4 Liu Jing (2002): Magnetorheological Fluids: From Basic Physics to Application JSME International Journal Series B 45(1): 55-60.
5 Boschke E., Steingroewer J. und Bley T. (2005): Einsatz der biomagnetischen Separation zur mikrobiologischen Qualitätskontrolle von Lebensmitteln. Chemie Ingenieur Technik 77(7): 912-919.
6 Berensmeier S. (2006): Magnetic particles for the separation and purification of nucleic acids Appl Microbiol Biotechnol 73:495-504.
7 Sieben S., Bergemann C., Lübbe A.S., Brockmann B., Rescheleit D. (2001): Comparison of different particles and methods for magnetic isolation of circulating tumor cells. J. of Magnetism and Magnetic Materials 225: 175-179.
8 Baumann S., Uta Ceglarek, Georg Martin Fiedler, Jan Lembcke, Alexander Leichtle, and Joachim Thiery (2005): Standardized Approach to Proteome Profiling of Human Serum Based on Magnetic Bead Separation and Matrix-Assisted Laser Desorption/Ionization Time-of-Flight Mass Spectrometry. Clinical Chemistry 51(6): 973-980.
9 Wadud S, Leon-Velarde CG, Larson N, Odumeru JA. (2010): Evaluation of immunomagnetic separation in combination with ALOA Listeria chromogenic agar for the isolation and identification of Listeria monocytogenes in ready-to-eat foods. J Microbiol Methods. 6. [Epub ahead of print]
10 Panneerseelan L, Muriana PM. (2009): An immunomagnetic PCR signal amplification assay for sensitive detection of Staphylococcus aureus enterotoxins in foods. J Food Prot. 72: 2538-46.
11 Mercanoglu Taban B, Ben U, Aytac SA. (2009): Rapid detection of Salmonella in milk by combined immunomagnetic separation-polymerase chain reaction assay. J Dairy Sci. 92: 2382-8.
12 Haukanes, B. I., Kvam, C. Application of magnetic beads in bioassays. Bio-technology 11(1), 60-63 (1993)
13 Safarik, I., Safarikova, M., Forsythe, S.J., (1995): "The application of magnetic separations in applied microbiology". The Journal of Applied Bacteriology 78, S. 575-585.

## Patentansprüche

1. Verfahren zur Abtrennung eines Analyten aus einer komplexen Probe mittels ferromagnetischer Partikel, welche eine ferromagnetische Legierung aus Mangan/ Zink Ferrit enthalten oder daraus bestehen, mit den folgenden Schritten:
a) Bereitstellung und Aufnahme der Probe in einer Flüssigkeit,
b) Bereitstellung von ferromagnetischen Partikeln, welche eine ferromagnetische Legierung aus Mangan/ Zink Ferrit enthalten oder daraus bestehen, mit einer mittleren Größe von 0,3-50 µm als magnetischer Partikel, an deren Oberfläche ein den Analyt bindender immobilisierter Ligand angeordnet ist,
c) in Kontakt bringen der Probe mit den ferromagnetischen Partikeln,
d) Einwirken lassen der in der Probe befindlichen ferromagnetischen Partikeln zum Binden des Analyten,
e) Anlegen eines Magnetfeldes,
f) Abtrennung der in dem Magnetfeld festgehaltenen ferromagnetischen Partikeln.

2. Verfahren nach Anspruch 1, wobei die Partikeln monolithisch sind.

3. Verfahren nach Anspruch 1 oder 2, wobei der ferromagnetischen Partikel eine mittlere Größe von 6-8 µm aufweist.

4. Verfahren nach mindestens einem der Ansprüche 1-3, wobei der den Analyten bindende immobilisierter Ligand beim in Kontakt bringen mit dem Analyten eine biospezifische Wechselwirkung mit diesem ausbildet.

5. Verfahren nach mindestens einem der Ansprüche 1-4, wobei der den Analyten bindende immobilisierte biospezifische Ligand ein Oligo- oder Polypeptid, insbesondere ein Antikörper, ein Antikörperfragment, eine Bindungsdomäne eines Enzyms, eine Nukleinsäurebindungsprotein, oder ein Lektin ist.

6. Verfahren nach mindestens einem der Ansprüche 1-4 wobei der den Analyt bindende immobilisierte biospezifische Ligand ein Oligo- oder Polysaccharid ist.

7. Verfahren nach mindestens einem der Ansprüche 1-4 wobei der den Analyt bindende immobilisierte biospezifische Ligand ein Oligonukleotid insbesondere eine DNA Sonde ist.

8. Verfahren nach mindestens einem der Ansprüche 1-4 wobei der den Analyt bindende Ligand eine ionische Gruppe, insbesondere eine Carboxy-, Sulfon-, Sulfat- Aminogruppe ist und beim in Kontakt bringen mit dem Analyten eine ionische Wechselwirkung mit diesem ausbildet.

9. Verfahren nach mindestens einem der Ansprüche 1-8, wobei der Ligand über eine Zwischenschicht bestehend aus einem quervernetzen Protein, einem polykondensierten Siloxan oder einem Polysacharid, insbesondere einem Aminozellulosecarbamat an den ferromagnetischen Partikel gebunden ist.

10. Verfahren nach mindestens einem der Ansprüche 1-9, wobei sich an den Schritt f) ein Schritt anschließt, bei dem der Analyt oder Teile des Analyten vom Partikel in eine abgetrennte Fraktion dissoziiert werden und für den Nachweis die abgetrennte Fraktion eingesetzt wird.

11. Verfahren nach mindestens einem der Ansprüche 1-10 wobei der Nachweis des Analyten am ferromagnetischen Partikel, durch Zugabe eines detektierbaren Konjugate erfolgt, das mit dem Analyten eine spezifische Bindung eingeht.

12. Verfahren nach mindestens einem der Ansprüche 1-11, wobei der Analyt ein Mikroorganismus, Teile eines Mikroorganismus, ein Virus oder ein Toxin aus einer klinischen Probe, einer Umwelt-, Lebensmittelprobe oder einer Probe aus einem pharmazeutischen Rohstoff oder Wirkstoffes ist.

13. Verfahren nach mindestens einem der Ansprüche 1-4, 7-10 wobei der Analyt eine in der Probe frei vorhandene RNA oder DNA ist.

14. Verfahren nach einem der Ansprüche 1-13, wobei die Abtrennung des Analyten mittels einer Vorrichtung mit mindestens einer ersten Kammer (1) und mindestens einer zweiten Kammer (2) erfolgt, wobei in der ersten Kammer (1) eine komplexe Probe (15), die gegebenenfalls in einer Flüssigkeit aufgenommen wurde, angeordnet ist und die Probe (15) mit ferromagnetischen Partikeln (10), an deren Oberfläche der den Analyt bindenden immobilisierte Ligand (20) angeordnet ist, mit einer mittleren Größe von 0,3-30 µm in Kontakt gebracht worden ist,
in der mindestens zweiten Kammer (2) eine flüssige Phase vorliegt und ein Magnetfeld durch einen in die zweite Kammer (2) tauchenden Magneten (3) auf die erste Kammer (1) einwirkt, wodurch die ferromagnetischen Partikel durch eine die erste Kammer (1) und zweite Kammer (2) trennende poröse Trennwand (4), die Poren aufweist, die Partikel mit einer Partikelgröße >100 µm zurück hält, in die zweite Kammer (2) eindringen und danach weiter prozessiert werden.
